# EUROPEAN PATENT SPECIFICATION

(11) **EP 4 534 016 B1**
(45) Date of publication and mention of the grant of the patent: **08.07.2026**
(21) Application number: 24205124.1
(22) Date of filing: 07.10.2024
(51) Int. Cl.: A61B 8/00

(54) **USER INTERFACE FOR ULTRASOUND IMAGING SYSTEM**
BENUTZEROBERFLÄCHE FÜR ULTRASCHALLBILDGEBUNGSSYSTEM
INTERFACE UTILISATEUR POUR SYSTÈME D'IMAGERIE PAR ULTRASONS

(30) Priority: 08.10.2023 US 202363588742 P; 08.10.2023 US 202363588738 P
(43) Date of publication of application: 09.04.2025
(73) Proprietor: BFLY Operations, Inc., Burlington, MA 01803 (US)
(72) Inventor: Thiele, Karl, St. Petersburg, 33701 (US)
(74) Representative: Kelly, Edward

(56) References cited:
- US-A1- 2020 320 694
- US-A1- 2023 200 776

## Description

### FIELD

The systems and methods described herein relate to medical imaging devices and aspects of the technology described herein relate to collection of ultrasound data along different elevational steering angles to generate improved image viewing features for users.

### BACKGROUND OF THE INVENTION

Scientists and engineers have made remarkable advances with medical imaging technologies, including imaging probes that are portable, provide facile use and ready transport and produce clinically excellent images. Such devices allow doctors and other clinicians to use medical imaging for patients who are remote from hospitals and clinics with imaging equipment or who treat patients who are confined to home. As such, these portable systems provide patients access to the excellent care that advanced medical imaging can provide. Examples of such portable imaging systems include those described in US Patent 10,856,840 which is granted to the assignee hereof. Other examples of portable imaging systems are known from document US 2020/320694 A1.

Although these systems work remarkably well, the demand for these systems extends to users who are less well trained than the typical sonographer. Today, sonographers are highly trained and often specialized in sonographic procedures such as imaging for abdominal, vascular, OBGYN, and echocardiography indications. These sonographers and other imaging professionals have learned excellent techniques for using and manipulating the ultrasound probe to collect images that are useful for the diagnostic effort or the procedure. Many of these techniques involve the careful maneuvering of the probe head across the habitus of the patient. These maneuvers distribute, orient and position the probe head in a way that collects a better, or more useful image for the treating clinician. Learning how to maneuver the ultrasound probe in this way is difficult. The ultrasound beam is, of course, invisible and this requires the sonographer to use indirect feedback as to the position and direction of the ultrasound beam. Often this feedback comes from watching the ultrasound image on a display. However, such images are often delayed in time from when the image was collected and they can be grainy and difficult for an unskilled user to decipher. In any case, getting visual feedback as the ultrasound image is being collected often requires the sonographer to develop a feel for how the image will appear on a display as the sonographers orients the probe head toward the anatomical target being imaged. The development of this feel can take quite a while to occur. Moreover, even when the skilled sonographers has developed the techniques for orienting the probe to capture images useful for the procedure, the technique developed by the stenographer may be somewhat individualistic and clinicians working with the images captured by different sonographers need to recognize that the collected image was taken using one type of technique versus another similar technique. For instance, some sonographers move the probe head across the habitus of the patient more quickly than other sonographers, and this can change certain characteristics of the collected images.

To extend the use of such instruments to a broader patient population who would benefit from the medical information provided by ultrasound devices, the devices need to become more facile to use. As such, there remains a need for systems that make the use of these sophisticated ultrasound imaging devices more facile for all the clinicians that can benefit from, and improve patient care, by the use of an ultrasound imaging system.

### SUMMARY OF THE INVENTION

The systems and methods described herein provide, among other things, systems for capturing images using ultrasound and in particular, systems that provide an ultrasound probe having a two-dimensional (2D) array of ultrasound transducers elements, typically capacitive micromachined ultrasound transducer (CMUT) elements that are arranged into an array. In one embodiment, the systems described herein leverage the 2D array of transducer elements to achieve beam steering over the generated ultrasound signals. The system applies beam steering to generate multiple ultrasound signals which are, in sequence, transmitted across the elevational dimension of the 2D transducer array and at different angles of orientation. Typically, the sequence of transmission is controlled so that the effect of the beam steering to achieve an imaging procedure comparable to a sonographer rocking a probe head about a location on the habitus of the patient. Each transmission in the sequence can be treated as a slice of a final image (whether still or video) that can be presented on a display. The final image is generated by image processing the multiple different slices of images to join the images slices together into a composite image. The joined image can be employed as a three-dimensional (3D) model of the anatomical target being imaged. Further, the joined images may be presented on a display to the user. Optionally, the joined images may be presented as a video of images made by sweeping the ultrasound beam over an anatomical target. As will be described herein, this can provide a useful image which highlights anatomical features of the image target.

As such, it will be understood that in one aspect the systems and methods described herein include methods for generating a three-dimensional (3D) model of an imaging target of an ultrasound scan. In one aspect, an ultrasound device having a two-dimensional (2D) flat array of micromachined ultrasound transducers can utilize beam steering to take a series of ultrasound images at a range of angles along an axis. The images may be collected, analyzed and processed to generate a 3D model of the subject matter from the series of images. The images are analyzed by applying a polar coordinate reference frame to each image in the series of images. The polar coordinate reference frame includes the imaging angle, which is the angle between an elevational dimension of the 2D array and the direction of ultrasonic wave travel. The polar coordinate reference frame also records the distance between a detected imaging target and the 2D array. By applying the polar coordinate reference frame to each image in the series of images and converting the polar coordinates to cartesian coordinates, the system can construct a 3D model of the imaging target. The system can then display, to a user, a view of the imaging target from a point of view other than the point of view at which the target was originally imaged. This allows a user to view the imaging target from multiple angles without changing the position of the ultrasound device. In some embodiments, the viewing angle of the imaging target would not be possible to achieve with an external probe. In such embodiments, the system and methods of the present application may, for example, display a 3D model of a kidney at an angle directly below the organ, which would require a physical probe to be inserted into the habitus of the patient to achieve.

More particularly, in some aspects the systems and methods described herein include ultrasound systems that include a handheld ultrasound imaging device including a flat two-dimensional (2D) array of micromachined ultrasound transducers (MUTs), and a processor configured to control the 2D array to take a series of ultrasound images along an elevational direction of the 2D array where each image is taken at a different angle relative to an axis parallel to the elevational direction of the array by beam steering ultrasonic signals produced by the MUTs. Typically the 2D array is rectangular and the elevational direction corresponds to the shorter dimension of the array. Further, the device includes a memory to store the series of ultrasound images as a series of ultrasound image data. Ultrasound image data includes information to generate ultrasound images on a display, such as the display on a smartphone or tablet, recorded in an electronic format suitable for storing in computer memory, processing by computer processing elements, and transmitting between electronic devices. Beam steering refers to a technique for controlling the direction of travel of ultrasonic signals by controlling the transducers to propagate an ultrasonic wave in a direction other than one perpendicular to the surface of the array.

Additionally, in some aspects, the systems and methods describe herein include a handheld computing device coupled to the handheld ultrasound imaging device having a memory capable of storing ultrasound image data, wherein the handheld ultrasound imaging device is configured to transmit ultrasound image data to the handheld computing device and store the data in the memory. The handheld computing device includes an image processor analyzing the ultrasound image data stored in memory to reconstruct a three-dimensional (3D) model of an imaging target, and a display for displaying ultrasound image data or the 3D model as any one of a still image, a video, or a cine. Typically the handheld computing device is any one of a standard smartphone or tablet, but those skilled in the art will know that any device suitable for the application will apply.

In some aspects, the image processor analyzes the ultrasound image data stored in memory by identifying an angle of imaging for each image in the series of ultrasound images, and identify elements of the imaging target within the ultrasound image. An angle of imaging refers to the angle between the direction of travel of the ultrasonic wave, and an axis parallel to the elevational dimension of the array of MUTs. Typically, elements of the imaging target are identified by edge detection methods to determine where, within the field of view of the imaging device, an anatomical structure first comes into contact with an ultrasonic signal. Depending on the imaging target, an anatomical structure and other elements may be identified by analyzing the relative timing at which ultrasonic signals propagate back into the array of MUTs.

In one embodiment, the image processor analyzes the ultrasound image data stored in memory to apply a polar coordinate reference frame to the ultrasound image data and converts the ultrasound image data into image data in cartesian coordinates. Applying a polar coordinate reference frame involves comparing an imaging angle of each image and a distance from the array of each element identified in the image and comparing the corresponding values in each image in the series of ultrasound images. This translates the ultrasound image data so that the elements imaged by the device can be described by their polar coordinates relative to the array.

In some aspects, the image processor is configured to select an image from the series of ultrasound images and designates it as a key-frame. The key-frame may be an image in the series of images where the system determines the imaging target, or a pathology within the imaging target, is advantageously clear. The image processor is configured to display the key frame as a default still image, or as a central frame in a displayed video or cine. Depending on the application at hand any one of the display options may be preferable.

In some embodiments, the system includes a user interface to allow the user to select a different image from the series of ultrasound images and designate it as the key-frame. In some applications the user may determine that a frame other than the frame selected by the system is preferable as the key-frame.

To create a 3D model of the target, in certain embodiments, the image processor is configured to join together the series of ultrasound images taken along an elevational direction of the 2D array to reconstruct the three-dimensional (3D) model of the imaging target as a model having data of the imaging target throughout a three-dimensional space.

In order to view desired aspects of the imaging target, the user interface may allow a user to manually pause, fast-forward, and rewind a displayed video or cine. Depending on the application at hand, a user may determine that viewing the ultrasound image data as a still image, a video, or on a short loop is preferable.

In some embodiments, the systems and methods described herein include a single button on the ultrasound imaging device to activate the processor to control the array. Depending on the application at hand the system may include a user interface configured to display the 3D model from a different angle than the angle of imaging. This provides the user with a different perspective view of the 3D model after one simple act.

In another aspect, the systems and methods described herein include a method of imaging an organ with a handheld ultrasound imaging device including a two-dimensional (2D) array of micromachined ultrasound transducers (MUTs), a processor capable of controlling the array. Further including, a handheld computing device including an image processor, where the handheld ultrasound imaging device takes a series of ultrasound images along an elevational dimension of the 2D array where each image in the series of ultrasound images has a different angle of imaging relative to an axis parallel to the elevational dimension of the array by beam steering ultrasonic signals produced by the MUTs. The processor of the ultrasound imaging device processes each image in the series of ultrasound images to generate ultrasound image data and transmits the ultrasound image data to the handheld computing device. The image processor processes the ultrasound image data to generate a three-dimensional (3D) model of elements imaged by the series of ultrasound images, and displays the ultrasound image data or the 3D model to the user.

The systems and methods described herein further provide, in one aspect, ultrasound imaging devices, whether portable, wearable, cart-based, PMUT, CMUT or otherwise, have controllers that have a real-time enhanced image display mode where the system may continuously sweep back and forth in the elevational dimension. Such a sweep feature may allow the user/clinician to survey the field faster and with more confidence to find areas of interest (e.g. b-lines, plaque, stones). As such, the imaging devices described herein with the enhanced image display includes a sweep function that may be understood, for purpose of illustration, as analogous to real-time b-mode, where the user slowly tilts the probe back and forth in the elevation direction. As discussed above, the systems and methods described herein may collect multiple image slices and join them into a single prospective volume acquisition to create a 3D model. These systems and methods may further provide a user interface sweep function that will employ the beam steering and multiple images slices to create an display image for the user that continuously "rocks" the plane back and forth in the elevational dimension, with a periodicity of approximately one to two seconds. In typical embodiments, the sweep function is not automatically selecting the planes (in the example herein there are seventeen planes) in real time. One example intended use is to facilitate the observation of b-lines. B-lines tend to be better observed when the interrogating ultrasound plane is perpendicular to the pleural lining. Since it is difficult to know when one is perpendicular to this surface, an experienced user will rock the probe back and forth in elevation. The user interface sweep function described herein performs this function for such a user, helping to achieve an image capture suitable for a clinician to use.

To this end, in certain embodiments, the sweep function will present the multiple image slices collected during this simulated rocking process, and the result, as these are b-mode images, is that the b-mode image with the better or more perpendicular orientation will appear more brightly within the image formed from the images captured while rocking. This creates an image display that is a real time and varying composite of the b-mode slices collected during a sweep back and forth in the elevational dimension. This can cause the important parts of the image to jump out to the clinician as they brighten up as the more perpendicular orientation is achieved.

In one aspect, the flat 2D transducer array which can beam steer the ultrasound signals to sweep across the elevational direction of the transducers, allows a less experienced user to hold the handheld probe still and essentially flat against the habitus of the patient while the probe executes the sweep function to take multislice images, which the system then displays to the users and builds into a 3D model of the imaging target.

### BRIEF DESCRIPTION OF THE DRAWINGS

The systems and methods described herein are set forth herein and, for purpose of explanation, several embodiments are set forth in the following figures.
FIG. 1 depicts one embodiment of the systems described herein;
FIG. 2 depicts pictorially a system having a controller for controlling imaging of a probe and for operating the probe in a sweep configuration;
FIG. 3 is a block diagram showing a probe, such as a probe of Fig 1, and how in the sweep mode the planes are swept in the elevational dimension;
FIG. 4 is an example image from a video generated of real-time B-Mode of a lung (between the intercostal spaces) showing radial lines that streak down from 3 to 12 cms;
FIGS. 5A and 5B depict a real-time image capture with the methods described herein, and depict in stills of a video from the lung imaged in FIG. 4, "b-lines";
FIG. 6 depicts three side-by-side examples of images captured with the methods described herein, the first being the carotid artery, the next being the vertebral artery and the third being the lung;
FIG. 7 is an example image of a static B-mode Image of a transplant right kidney.
FIGS. 8A and 8B depict a multislice cine captured using the techniques described herein and with the process depicted in Fig. 3, and shows a kidney from upper to lower pole and in this example hydro was identified in the upper and mid poles;
FIG. 9 depicts a process wherein the user can select a keyframe they wish to use.
FIG. 10 depicts a process wherein ultrasound image data store in memory is used to produce a 3D model;
FIGS. 11A, 11B and 11C depict ultrasound images collected at an early, middle, and late portion respectively of a multislice scan through a kidney and each image depicts a different cross sectional view of the kidney as the multislice scan images from one pole to the opposite pole along the elevational direction of the array;
FIGS. 11D, 11E, and 11F depict 3D reconstruction of a multislice scan of a kidney and FIGS. 11D, 11E and 11F use the same view as the angle of imaging such that the reconstruction in FIGS. 11D, 11E and 11F generally correspond to the images in FIGS. 11A, 11B and 11C;
FIGS. 12A, 12B and 12C depict the images from the multislice scan depicted in FIGS 11A, 11B and 11C and the outline of the kidney is identified;
FIG. 13 depicts an ultrasound image from a multislice scan and 3 views of a 3D reconstruction generated from the scan with each view of the 3D reconstruction from different angles in the X, Y, and Z dimension; and
FIG. 14 depicts an ultrasound probe in contact with a subject and imaging a target within the subject.

### DETAILED DESCRIPTION

In the following description, numerous details are set forth for purpose of explanation. However, one of ordinary skill in the art will realize that the embodiments described herein may be practiced without the use of these specific details. Further, for clarity, well-known structures and devices are shown in block diagram form to not obscure the description with unnecessary detail.

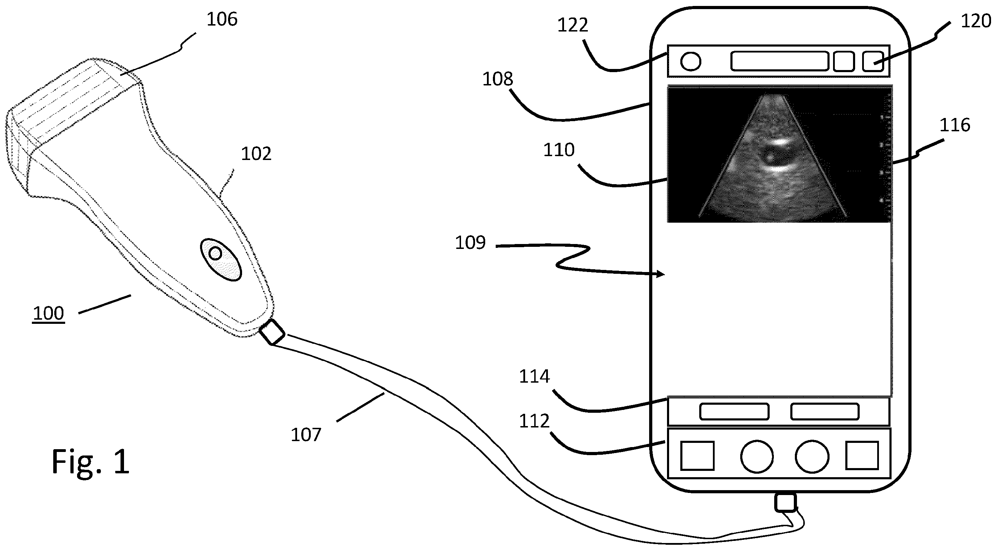

In one embodiment, the systems and methods described herein include, among other things, the system 100 depicted in FIG. 1. The system 100 depicted in Fig. 1 is a handheld ultrasound probe of the type that can connect to a smartphone or a tablet to display ultrasound images, including still images and video, to a clinician. Although the systems and methods described herein may be used with cart-based ultrasound system and other ultrasound imaging systems, for purposes of illustration, the systems and methods described herein will be described with reference to ultrasound handheld probe systems such as the system 100. These handheld systems are more portable and less expensive than traditional cart-based ultrasound systems and as such provide the benefits of ultrasound imaging to a larger population of patients. One such portable handheld system is the iQ series of ultrasound probe systems manufactured and sold by the Butterfly Network Company of Burlington Massachusetts.

As will be described herein, the portable system 100 of Fig. 1 includes a user interface feature that allows the user to place the probe 100 (in this case the probe head 106) against the patient and sweep through a sequence of imaging processes that collect a sequence on image slices. The system 100 will join the image slices to create a 3D model of the anatomical target imaged with probe. The system 100 will allow the user to view that 3D model of the anatomical target from alternate perspectives. Additionally, the image slices may be presented as a sequence of images that are presented in a video format and allow the user to see the different image slices in sequence. Typically, but optionally, the system allows the user to select an image frame from the presented video to act as a key frame, typically selecting the image frame that most clearly presents the anatomical feature of interest.

To this end, and to illustrate such a system in more detail, FIG. 1 depicts the system 100 that includes a probe 102 and a handheld device 108. The probe 102 has a transducer head 106. The probe 102 is coupled by a cable 107 to a handheld device 108, depicted in FIG. 1 as a mobile phone. The depicted handheld device 108 is executing an application that collects data, including image data, from the probe 102. The application may display a live and moving image within the image window 110 and may display within software UI windows, such as the software UI window 112, one or more controls for operating the probe 102, such as a control for enabling sweep operation that will direct the probe 108 to take a sequence of image slices across the elevational dimension of the transducer array and to join the image slices into a single model that can provide a 3D model of the anatomical target. Additionally, the user interface controls in window 112 may allow the user to present the image slices on the display of the handheld, such as depicted by image 116, to show the sequence of image slices collected by the transducer head. In FIG. 1 the system is a handheld probe, but as noted above this sweep mode can easily be extended to a cart-based system.

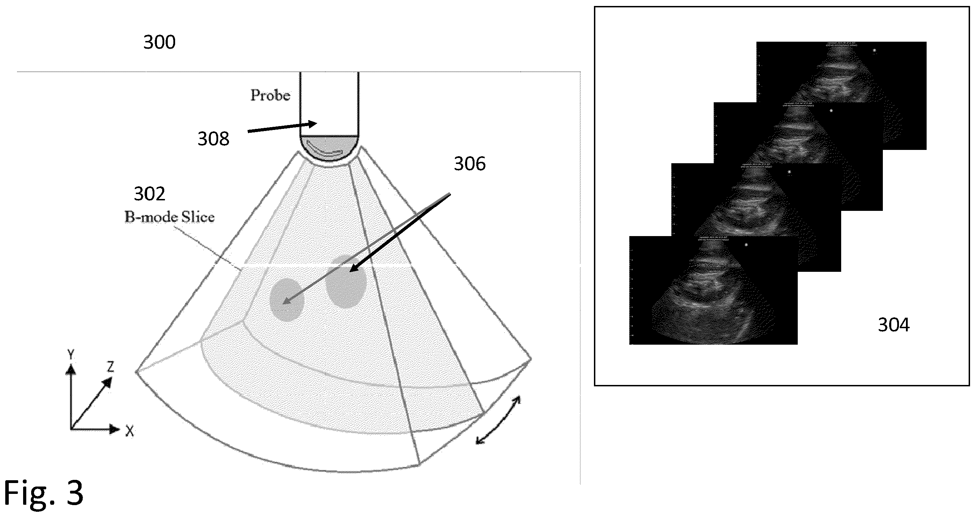

The probe 102, in this example embodiment, is an ultrasound probe of the type disclosed in US Patents 10,856,840 and 11,167,565 both assigned to the assignee hereof. The probe 102 is a handheld ultrasonic imaging probe that can be used by the clinician to image a patient and collect medical images useful in the clinical process of diagnosing and treating the patient. The probe 102 has a transducer head 106 that the clinician may place against the tissue of the patient, such as by placing the transducer head 106 in contact with the patient's chest proximate to the heart or lungs of the patient or proximate the kidneys, or wherever the clinician wishes to image. In typical operation, the clinician uses a UI control button as a UI by which the clinician may activate various functions such as image capture operations that cause the application 109 executing on the handheld device 108 to store one of the images, such as depicted in FIG. 3, generated by the transducer head 106. That application 109 may render the captured images in the image window 110 for the clinician to view. In the sweep mode the images (the image slices) may be presented as if the probe were being physically rocked back and forth, rather than looped through the images.

The UI window 112 may provide the clinician with a series of optional user interface controls that the clinician may use to operate the application 109 executing on the handheld device 108 to change how the captured image is rendered, store the image, mark the image, and perform other types of operations useful during the tomographic procedure.

During a tomographic procedure or other imaging process, the clinician adjusts the position and angle of the probe 102 until an image of interest appears in the image window 110. In some embodiments, the clinician may activate the UI control button to capture images to study, or activate various functions such as the sweep/slice mode.

In the embodiment depicted in FIG. 1, the handheld device 108 is a programmable device that runs the application 109 that, for example, performs the image display functions, user interface functions, such as allowing the clinician to select presets and capture images from the image stream, and configures the system 100 with any selected preset parameters. In this embodiment, the handheld device 108 may be a smart phone, a tablet, or any other suitable handheld device capable of running application programs and of supporting a data connection to the probe 102. In the depicted embodiment the handheld device 108 couples to the probe 102 by way of the cable 107. However, in alternative embodiments, the handheld device 108 and the probe 102 may have a wireless connection of the type suitable for transferring data and control signals between the two devices. In one example, the wireless connection may be the Bluetooth protocol (IEEE 802.15.1), ultra-wideband (UWB, over IEEE 802.15.3), ZigBee (over IEEE 802.15.4), or Wi-Fi (over IEEE 802.11) connection or a connection using some other protocol for short-range wireless communications, preferably with low power consumption. However, any suitable wireless technology may be used including those that work with narrow bands, employ optical communication, or which use some other suitable technique for exchanging information between the probe 102 and the handheld device 108.

In the embodiment depicted in FIG. 1 the transducer head 106 includes an array of ultrasonic transducer elements. The array of ultrasonic transducers may be a 2D array of MEMs transducer devices, such as an array of capacitive micro-machined ultrasonic transducers (CMUTs) or an array of piezoelectric micromechanical ultrasonic transducers (PMUTs), that are capable of generating ultrasonic waves, including beamformed ultrasonic waves and detecting ultrasonic waves as they return from the patient. In one embodiment, the depicted transducer head 106 includes thousands of transducer elements that operate in coordinated action to create the ultrasound beam used for the image collection. In one example, the transducer head 106 includes a two-dimensional array of thousands of transducer elements organized into a 2D array and formed on a semiconductor die or chip. The die or chip may, in certain embodiments, further contain on-chip processing circuitry including more than one thousand analog-to-digital converters and amplifiers. Embodiments of transducers formed on a semiconductor die or chip are shown in more detail in US Patent 9,067,779 and in US application US2019/0275561. Embodiments of on-chip processing circuitry are shown in more detail in US Patent 9,521,991. In other embodiments, the transducer head may use PMUTs and the A/D converters and amplifiers may be on separate chips or dies and the chips and dies may be mounted on a circuit board or boards.

In operation, the transducer head 106 detects ultrasonic waves returning from the patient and these waves may be processed by processing circuitry formed on the same chip as the transducers, a signal processor, a CPU, an FPGA, or any suitable type of processing device, or any combination thereof, which may process the returned ultrasound waves to construct image data. That image data may be used by the application 109 running on the handheld device 108 to create images for the clinician.

In the depicted embodiment, the executing application 109 may include and image processor that processes the collected image slices to join the image slices together into a 3D model that can be stored in a data memory and presented in a display. The presentation of the joined image can be as the constructed images, including video images, such as the ultrasound image cine 116, on the image window 110 so that the clinician can see images of the patient as those images are being generated by the probe 102 or when the physician wishes to review the captured images. In the depicted embodiment, the application 109 also provides a UI window 112 that has a series of software control buttons, sometime called widgets, that the clinician may use for controlling the operation of the probe 102. These controls allow the clinician to change how images, such as image cine 116 are rendered, captured, and displayed on the handheld device 108.

In the embodiment of FIG. 1, the application 109 further has a menu 114 that depicts a preset selected by the clinician and an indication of the view selected for the clinician.

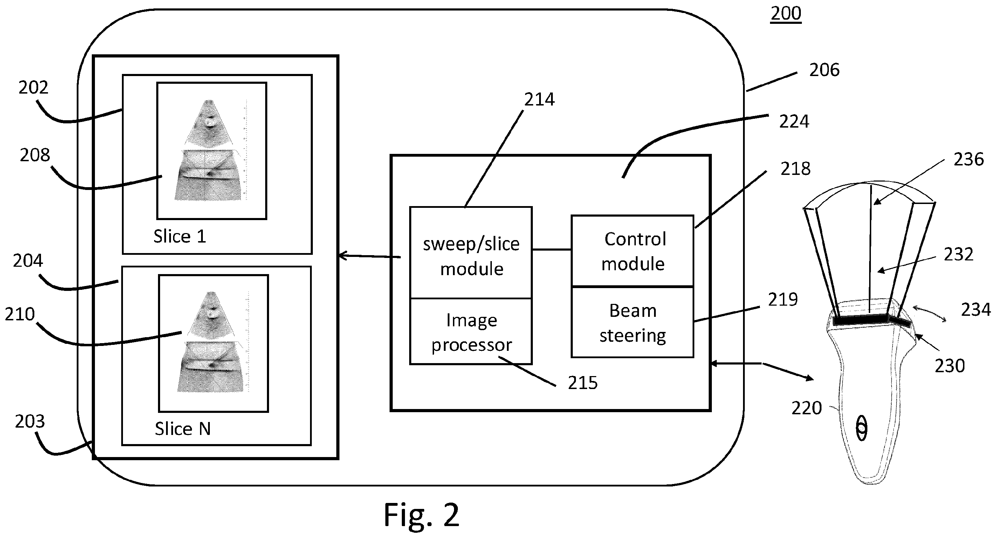

In one embodiment, the application 109 implements a sweep feature of the type depicted pictorially in FIG. 3. FIG. 2 depicts pictorially and in more detail a system 200 that has a handheld device 206 (such as a smartphone) executing an application 224 that may configure the system 200 to carry out the sweep/slice feature described herein. As will be described in more detail below, the user, typically the clinician taking the image, can employ the user interface of the system, such as by selecting a widget from the software UI window 112 that will put the probe and system into a mode to take multiple image slices in a sequence of image slices that sweep across the elevational dimension of the transducer array of the probe. Typically, in this sweep/slice mode the clinician will place the probe on the habitus of the patient at a location the clinician deems correct for imaging the anatomical target of interest, such as the patient's kidney. The clinician can place the probe so that the probe is essentially "flat" against the habitus of the patient. In this orientation the transducer array of the probe is such that an axis extending outward from the array and perpendicular to the array will extend into the patient and toward the anatomical image target of interest. In this sweep/slice mode the clinician will hold the probe steady so that the axis continues to extend toward the anatomical target of interest. The sweep/slice mode will operate the probe to, during a time period of 1-5 seconds for example, take a series of still images, each at a different, and typically contiguously progressing angle relative to the axis extending from the transducer array. In one example, the sweep/slice moder will capture about 45 still images, each at a different elevational angle and collectively sweeping through an arc of about 20 degrees to either side of the axis extending outward from the face of the transducer array.

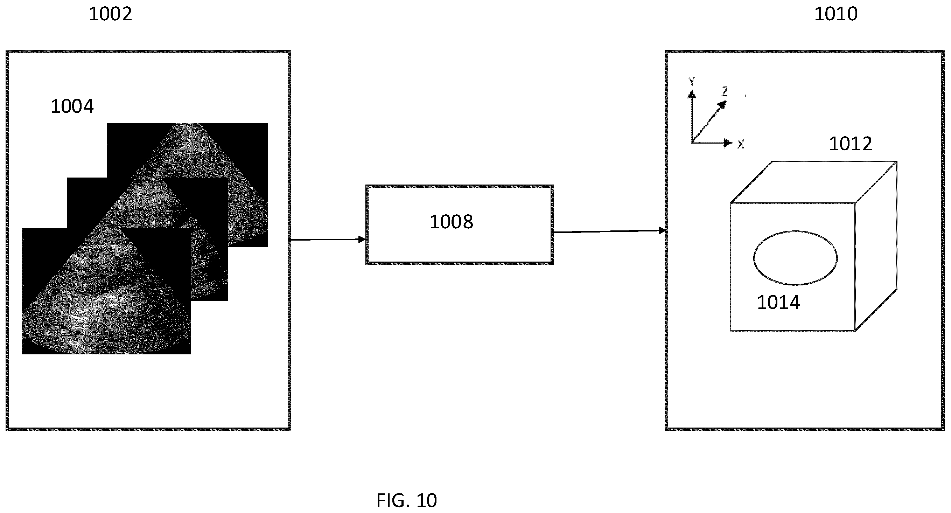

As described below, in particular with reference to Fig. 10, the system can join the different still image slices together to build a 3D model of the area imaged and provide the clinician with an interface tool to allow the clinician to view the model from different perspectives. Additionally, as described below, the user interface may allow the clinician to select an auto fan mode that will sequentially present a portion of the images to simulate viewing the anatomical area as fi the clinician were manually fanning or rocking the probe during imaging.

To this end and to implement these functions such as sweep/slice mode, auto fan, and building the 3D model, the system 200 includes an application 224 that typically is a computer program executing on a processor built within the circuit board of the handheld device 206. For clarity and ease of illustration, the application 224 is depicted as a functional block diagram and as an application running on the handheld. In alternate embodiments, the application can be run, at least in part, on the cloud and data stored on the cloud.

The functional blocks of application 224 include a control module 218, a beam steering module 219, a sweep/slice module 214 and an image processor module 215. The handheld device 206 also includes a video module 203. The video module 203 can handle video tasks, such as capturing streams of video data generated by the probe 220 and rendering the video on the display of the handheld device 206. In one example, the video module 203 converts the collected images 208 and 210 generated by sweep/slice mode module 214 that in one embodiment will allow the user to implement the auto fan presentation that simulates manually continuously "rocking" the image plane back and forth in the elevational dimension, with a periodicity of for example approximately 1 to 2 seconds, 3 to 5 seconds, or whatever time period is suited for the application, such as kidney imaging. As shown in Fig. 2, the video module 203 includes image memory 202 and 204 that can store respective image data for each slice, such as the depicted image slices 210 and 208. The image slice image data 208 and 210 is the data collected by the beam-steer-controlled array 230. The number of image slices taken may differ depending upon the simulated rocking process (how much of a rocking angle and how long the rocking takes) and the periodicity may vary. For example, it may vary depending upon the preset being used, the anatomical organ being imaged, the depth of the imaging and other parameters. Additionally, the user may have some control over the periodicity and may change the period based on watching the image and selecting a period that causes features in the live image from sweep mode becoming more clear to the user within the display. In Fig. 2 only two image slices are shown, but it will be understood that the sweep/slice module 214 typically takes more image slices, and for example taking 40 to 50 image slices during a sweep/slice operation is common.

Fig. 2. further depicts that the handheld device 220 includes a 2-dimensional (2D) array of ultrasound transducers 230. Fig. 2 depicts the array 230 using perforated lines as the array 230 is typically encompassed and covered by an acoustically transparent housing made of a biocompatible material that is suitable for contacting the habitus of a patient. Fig. 2 further depicts that the transducer array 230 is operated by the control module 218 and the beam steering module 219 to generate an ultrasound signal that is angled relative to the face of the transducer array 230. In one embodiment, the control module 218 generates operating parameters that can be provided to the 2D array 230 to control operation of the array 230 and typically to separately control each transducer in the 2D array 230. Parameters can include frequency of operation, duration of operation, timing, and therefore relative phase of operation, and other parameters relevant to operation of the array 230. In one embodiment, the array 230 includes or is connected to an ASIC or microcontroller that can collect operating parameters from the control module and load them into the ASIC in a manner that will allow the parameters to control operation of the individual elements of the transducer array 230. The beam steering module 219 in one embodiment generates a beam steering parameter for the control module 218 to deliver, with other operating parameters, to the transducer array 230. The beam steering module 219 can generate the parameters needed to sweep the ultrasound beam 232 across the elevational angle 234 of the transducer array 230, and take the number of image slices suited to the application, such as 40-50 image slices across for example a 20-30 degree arc about an axis 236 perpendicular to the face of the array 230.

Fig. 2 depicts one such ultrasound signal, 232, which represents an ultrasound signal employed to generate one image slice. In this embodiment, the beam steering module 219 will direct the handheld device 220 to operate the 2D transducer array 230 to generate multiple image slices, such as 10 to 20 image slices, 20 to 30 image slices, 40 to 50 image slices or which number of slices is the suitable number of image slices for the application at hand. In any case, it can be seen from Fig. 2 that the image slices are to be each directed along a different elevational angle by using the beam steering module 219 to control, typically, a phase shift that is applied to the ultrasound signals generated independently by respective ones of the transducer elements within the 2D array of transducer elements 230. Techniques, for phase shifting an array of transducers for transmitting and receiving ultrasound signals are known in the art, and any suitable technique for such beam steering may be employed. Such techniques are disclosed in for example, Kinsler et al.; Fundamentals of Acoustics; John Wiley and Sons, pages 188-204 (2000); and Shi-Chang Wooh, Yijun Shi; Optimum beam steering of linear phased arrays, Wave Motion, Volume 29, Issue 3, 1999, Pages 245-265.

Optionally, the systems and methods may use beam steering and adjust the aperture of the array 230 during sweeps across the elevational arc 234. To this end, the beam forming module 219 may configure the array 230 for successive iterations of transmitting and receiving ultrasound waves. Each set of ultrasound data collected from the successive iterations of transmitting and receiving ultrasound waves may be focused at different elevational steering angles using beamforming. The different elevational steering angles may be measured relative to the axis 236 extending outward and perpendicular to the face of the ultrasound transducer array 230. The beamforming process implemented by module 219 may include applying different timing/phase delays to the transmitted and received ultrasound waves/data from different portions of the ultrasound transducer array 230 such that there are different delays for different elevational rows, where a row refers to the transducer elements spaced along an line extending along the long axis of the ultrasound transducer array. Similar delays may be applied to all elements in a row, or each element in a row, or each element in the array 230, may have a separately determined delay. The technique used will depend on the application. Optionally, and as disclosed in US Patent 11,167,565 assigned to the assignee hereof, the aperture of the array 230 may vary depending, in part, on the elevational steering angle to address differences in signal-to-noise ratio for the ultrasound data collected when the steering angle for the data is different from a zero elevational steering angle. In this embodiment, the signal-to-noise ratio at more extreme elevational steering angles may be improve, typically increased, by transmitting ultrasound waves from and receiving ultrasound waves with a larger elevational aperture of the ultrasound transducer array 230 (i.e., by using more elevational rows). In this embodiment, the beamforming module 219 may vary the elevational aperture during an elevational sweep as a function of elevational steering angle. In particular, the elevational aperture may be increased with more extreme elevational steering angles. In some embodiments, the number of elevational rows used at different iterations of transmitting and receiving ultrasound waves during the sweep may vary for example, between approximately 2 and 64.

In operation, the user may hold the probe 220 in place against the habitus of the patient and the beam steering module 219 will generate, in response to a user interface command entered by the user, ultrasound signals, such as the depicted ultrasound signal 232, each of which is shifted along the elevational direction 234 to sweep the image slices 232 in an arc 234 across and over the elevational direction of the transducer array 230. This coordination of the sweep/slice module 214 and the control module 218 and the beam steering module 219 provide automated sequential ultrasound capture mode on the handheld device 220, by having the control module 218 send the appropriate parameters to the transducers elements of the array 230 as directed by the beam steering module 219 to thereby automatically steer the beam 232 to scan an organ and capture multiple ultrasound image slices at one time and across a wide angle. The sweep/slice imaging mode is designed to make it easier and faster to acquire excellent images without skilled maneuvering. These image files 208 and 210 generated and stored in memory 203 can either be immediately read and measured at the bedside by skilled scanners or, for those less experienced, can be sent to a specialist for further review, similar to the workflow of a CT or MRI. Additionally, as noted above, the system 200 provides a tool, for example for use with lung imaging, that allows users to capture and view real-time back-and-forth virtual fanning, making it easier to visualize for example A-lines and other lung pathology.

The development of applications such as the application 206 with the sweep module 218, the beam steering module 219 and the video module 203 that execute on a handheld device such as a smartphone or tablet and that carry out the depicted functions of the application 206 is well known to those of skill in the art. Techniques for developing such applications are set out in for example, Alebicto et al., Mastering iOS 14 Programming: Build professional-grade iOS 14 applications with Swift 5.3 and Xcode 12.4, 4th Four ed.; Packt Publishing Ltd (2021).

In optional embodiments, the systems described herein may be responsive to the type of imaging operation that the clinician is undertaking. In certain imaging devices, such as those described in US Patent 10,709,415 assigned to the assignee hereof, the probe 102 may be placed into a preset, such as one for Kidney. Table 1 below lists certain example presets.

**Table 1**

| |
|---|
| Preset |
| Abdomen |
| Abdomen Deep |
| Aorta & Gallbladder |
| Bladder |
| Cardiac |
| Cardiac Deep |
| Coherence Imaging |
| FAST |
| Lung |
| MSK-Soft Tissue |
| Musculoskeletal |
| Nerve |
| OB 1/GYN |
| OB 2/3 |
| Ophthalmic |
| Pediatric Abdomen |
| Pediatric Cardiac |
| Pediatric Lung |
| Small Organ |
| Vascular: Access |
| Vascular: Carotid |
| Vascular: Deep Vein |

Each preset is a mode adapted for a particular type of imaging study. Presets may help with imaging studies and may be employed within systems that have the sweep feature described herein.

FIG. 3 shows in more detail how the probe sweeps the ultrasound beam across an anatomical area. The probe 308 is positioned on the patient and collects B mode images slices that can be used by the image processor 215 to construct a 3D model of the anatomical area being imaged. The probe 308, as described above, may be operated in a way that beam steers across the elevational direction to effectively rock back and forth while taking an image periodically such as once every one or two seconds. In one embodiment, the probe travels through about 20 degrees of motion typically ±10 degrees above the center angle (that is relative to an axis extending outward and perpendicular from the face of the transducer array of probe 308). In the example depicted in Fig. 3, the probe 308 is collecting B Mode image slices. The B mode image slices collected during this rocking include B mode image slices such as depicted B mode image slice 302. These B mode image slices can be uploaded to a memory such as the memory 304 that will store the images as image data.

The sweep/slice module 214 depicted in Fig. 2 can collect the images and in cooperation with the video module 203 present them to the user in response to an auto fan mode selection made by the user through the user interface 112. In auto fan mode a live image is presented to the user on the handheld device. In one embodiment, the sweep/slice module 214 in cooperation with the video module 203 will present the images as if they are simulating a rocking maneuver of a trained sonographer, that is by passing through the images in one direction and then passing through them in the other direction. In other words, the images are displayed sequentially from a first angle to the furthest angle and then from that furthest angle back to that first angle. This is different than looping through the images. The bounced image or yo-yo image are brightness mode images so features of interest to the clinician will from time to time appear more brightly within some of the slices. By presenting them as a sequence of images, certain bright features will appear and naturally the display will have them appear more prominently and more easily observable by the clinician. This provides a real time sweep feature that can cause certain features of the anatomy to appear as an enhanced image on the screen one that has features of interest highlighted more brightly than others.

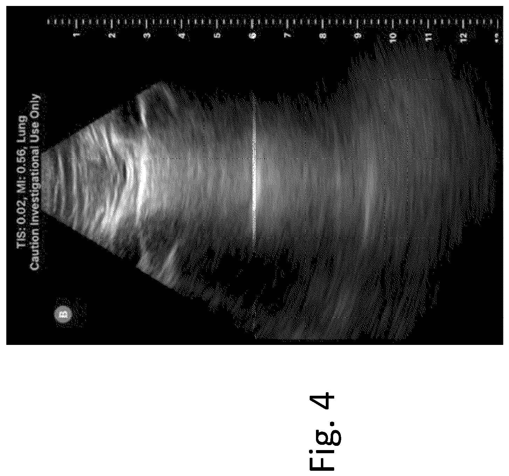

FIGS. 4 -6 depict actual example images and videos of the type that are captured and presented to users with the systems and methods described herein. Specifically, Fig. 4 presents an actual example of a single image slice from an actual live image taken over a time period of a few seconds, during which a system of the type described herein took multiple image slices. The image of Fig. 4 is a single image slice showing a B mode image. In particular, FIG. 4 is a real-time B-Mode of a Lung (between the intercostal spaces). As a real-time b-mode image it is a live image much like a video or gif. In this video shown by a single image in FIG. 4, one can see B-lines (radial lines that streak down from 3 to 12 cms). B-lines may be indicative of fluid in the lung, and can be used to diagnose Congestive Heart Failure, Pneumonia, Covid and other indications. Such images provide some information to a clinician but certain features may be more difficult to see because of the particular slice and the orientation of that real time live image slice relative to features of interest to the clinician.

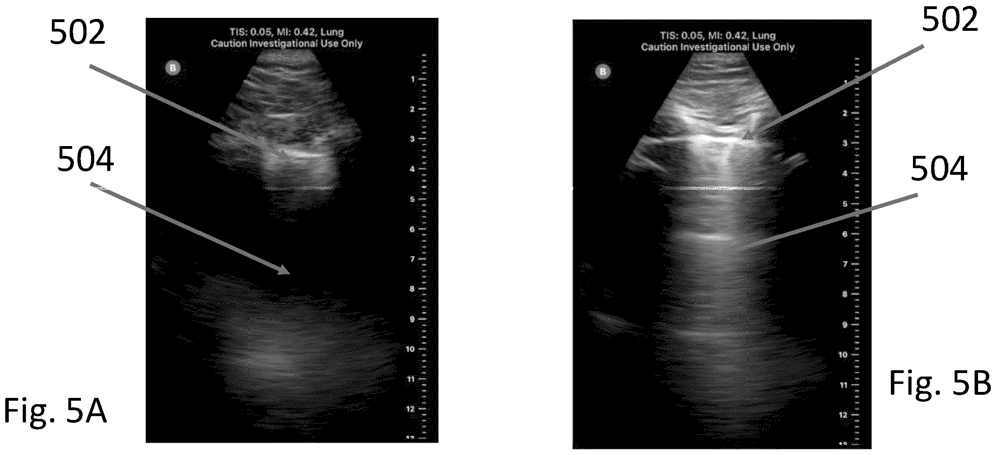

In contrast FIGS. 5A and 5B depict a live image that is being rocked during an auto fan display through the defined range of angles collected by the sweep/slice module. It will be noted, that for the purpose of preparing this disclosure, video and live images may not be presented fully, and FIGS. 5A and 5B merely represent such a live or moving image by presenting two screenshots of that moving image. But it will be understood by those of skill in the art that this image is actually a live or moving image; one that is generated by the sweep/slice module passing through and presented by video module 203 on the display, in sequence, the different images collected by the probe 308 while the probe 308 takes images as it steers the ultrasound beam through different elevational angles to simulate rocking back and forth between a first angle and a second angle.

In any case, FIGS. 5A and 5B depict ultrasound images collected from the same lung as at the same time as shown in FIG. 4, but in sweep/slice mode. One can see that in sweep/slice mode the image will change and different features such as the depicted feature 502 and the depicted feature 504 will appear as the image is presented as being rocked from the first angle to the second angle and back again. This allows certain features of interest to the clinician to appear more clearly and be more easily recognized by the clinician.

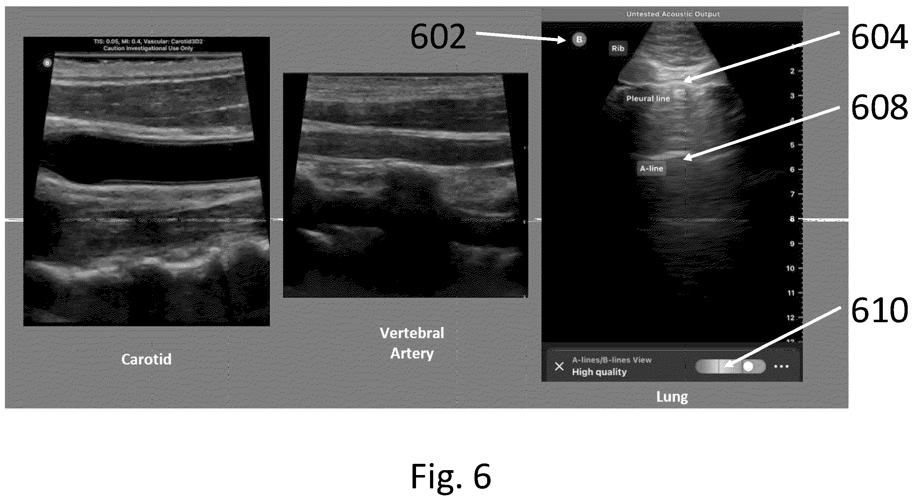

FIG. 6 depicts three side by side examples of images captured with sweep/slice mode, the first being the carotid artery, the next being the vertebral artery and the third being the lung. Again, although FIG. 6 presents the sweep/slice mode moving images as still images it is to be understood by those of skill in the art that all three are moving images, like gifs, or bouncing live images of an smartphone display, and made from a composite of slices taken by simulated rocking of the transducer back and forth by about 20 degrees about the center angle. It will be understood to those of skill in the art that the simulated rocking may be carried out by software directing the beam by controlling the transducers in the ultrasound head to achieve a selected angle.

Further, FIG. 6 shows an image on the right hand side of FIG. 6 of a sweep mode image that presents the B mode images being rocked back and forth to provide an enhanced display to the clinician. Additionally, this right-hand image is further enhanced to display annotations and includes indications of where the patient's ribs are located 620, the pleural line 604 of the patient and an A line 608 within the lung of the patient as well. On the lower right-hand side an widget 610, in this example an oval shape with a circle in it, indicates where within the angular sweep of the current image occurs. In operation the circle within that oval section widget 610 can move back and forth indicating that the live image is formed from a composite of the slices taken by rocking the transducer back and forth during imaging. The location of the circle within the oval indicate the relative angle of the slice currently being displayed.

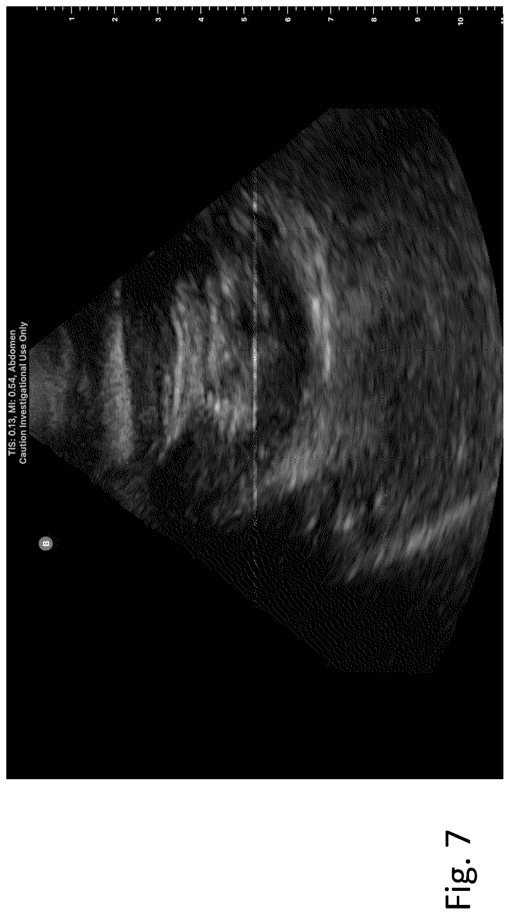

Returning to FIG. 3, it is noted that FIG. 3 shows the probe 308 taking multiple B-mode slices with one B-mode slice depicted individually as the slice 302 and the memory 304 storing actual examples of B mode image slices. The B-mode image slices can be collected and stored by the system 100 in memory such as in cloud storage memory and this is shown by the cloud storage 304 which stores the multislice images. Each individual slice may show some information about the anatomical feature being imaged, but collectively a cine may be run as a back and forth traveling sweep image that is looped and this can provide more comprehensive review of the anatomical organ being imaged. For example, in the actual examples of FIGS. 3, 7 and 8A and 8B, a kidney is being imaged. The probe can take B-mode slices 302 from one pole to the other pole. Any particular slice, such as that depicted in FIG. 7, may fail to show the collection of water within the kidney. However, operating the system so that the image presentation is in cine mode using the multislice images to generate a cine, renders the presence of hydro/water within the kidney more apparent and more easily viewable by the clinician. Thus, the B slice image depicted in FIG. 7, may not present the collection of hydro/water within the kidney in a way that is readily apparent to all clinicians.

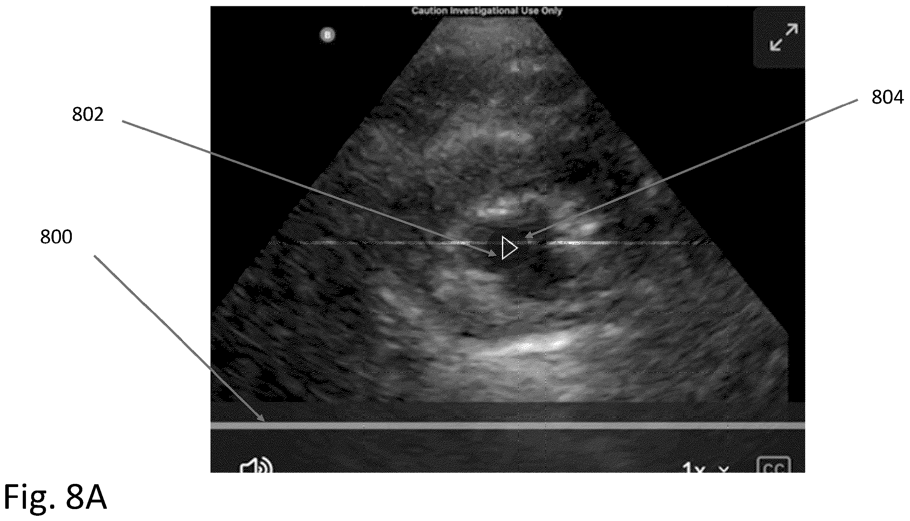

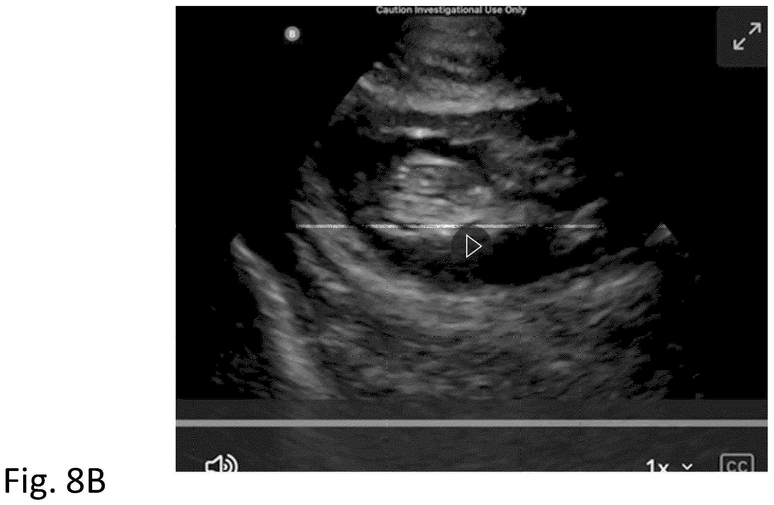

In contrast FIGS. 8A and 8B, (which are only two screenshots of the cine developed and presented to the user by the systems and methods described herein) present a cine loop of multislice images moving from the superior pole to the inferior pole of the kidney. That FIGs. 8A and 8B present a video cine is apparent from the time bar 800 and the play arrow 802. It further can be seen that the image in FIG. 8A includes a feature 804 that is not readily apparent in FIG. 7. The feature may grow more apparent as the cine loops, such as in FIG. 8B. Even though FIG. 7 is a B slice image of the same kidney, it is FIGS. 8A and 8B that actually to present more information, or at least different information, to the clinician. With this image and visual context provided by the cine, the clinician is much more able to identify the presence of hydro within the kidney.

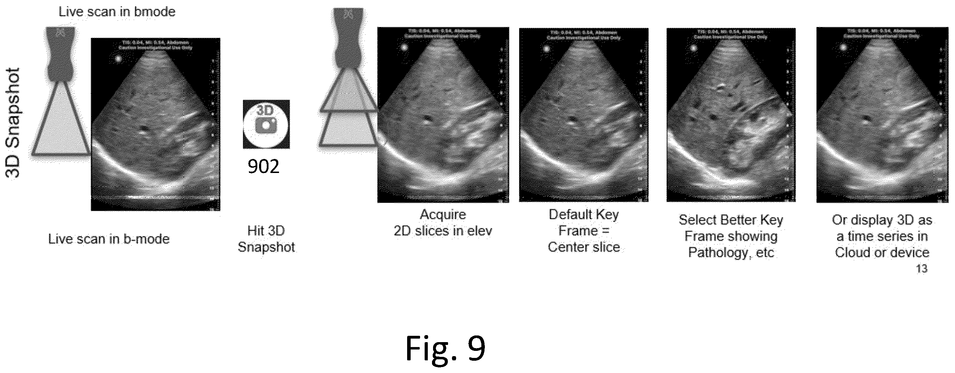

FIG. 9 depicts the 3D snapshot function and presents pictorially the probe taking a 3D snapshot in a live scan in B mode. The operator can use a 3D snapshot button 902 depicted graphically in FIG. 9 to pick a snapshot from the collected image slices. The snapshot button 902 can be a widget that appears in the UI window 112 on the handheld device. The snapshot button 902 widget can, while the system is presenting the user with the image slices, and allow the user to activate the snapshot button 902 to select one of the image slices to be the key frame. The key frame can be any frame chosen by the user, and typically will be the image slice selected by the user to present the pathology or other condition that the clinician wants visualized from the sweep/slice operation. In one practice, the sweep/slice module 214 will collect multiple image slices and the image processor 215 can generate a 3D model of the imaged anatomical target from the captured sequence of image slices. Optionally, during the sweep/slice process, the image processor 215 selects a default key frame, typically the image slice at the center of the sweep (essentially the image slice generated from the ultrasound beam directed perpendicular to the face of the transducer array 230), or allows the user to select a better or different key frame showing the pathology. Alternately, the snapshot button 902 may display the 3D image that gets created by the image processor 215 by bringing down the information from the cloud or the device and presenting it to the user. The user can repeat this process until the preferred key frame is successfully collected. In contrast today a 3D image is taken with a live scan in B mode then one hits the free frame or snapshot and sometimes stores frames to the cloud that are less clear as to the indication of interest to the treating physician.

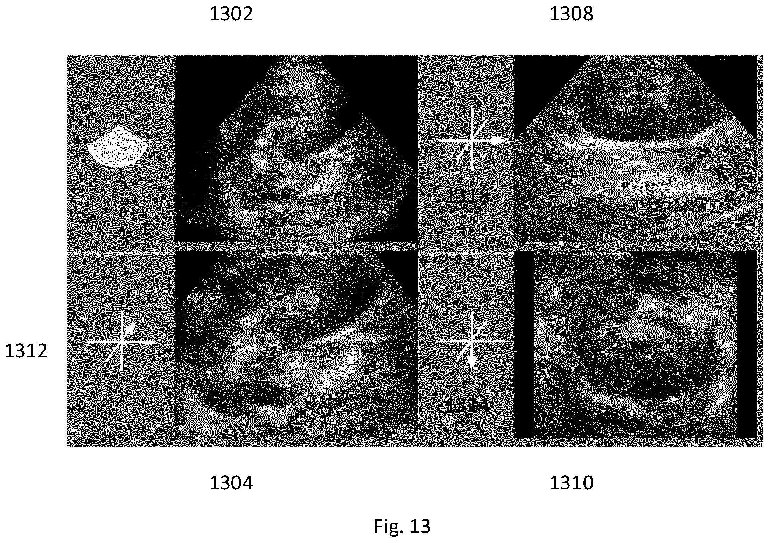

FIGS 10 depicts a diagram of an image processor system for generating a 3D model from a series of multislices. In typical embodiments, the systems and methods described herein create a 3D model of the target by applying an image processor that is configured to join together the series of ultrasound images taken along an elevational direction of the 2D array, which are 2D images, to reconstruct or actually generate a three-dimensional (3D) model of the imaging target as a model having data of the imaging target throughout a three-dimensional space. In this way a true 3D model of the imaging target is created. This allows the system to generate for the user views of image slices of the imaging target from different perspectives. Thus, the system can present simulated image slices to the user that present what the user would have seen if the image slice was captured by the probe from the new and different perspective. Thus, this 3D model allows the system to generate simulated image slices based on actual 2D images slices, captured from one perspective, but generated by the system to show an image slice, taken from the 3D model, from a perspective different from the perspective used to capture the 2D images slices. Fig. 13 depicts actual examples of simulating image data, typically image slices, based on a 3D model incorporates the captures 2D image data to construct a 3D model that contains imaging data across the three dimensions x, y and z.

The computer memory 1002 stores a series of multislice images as ultrasound image data 1004. The ultrasound image data 1004 is processed by the image processor 1008 which generates a 3D model 1012. The ultrasound image data is the sequence of image slices (still frames) collected as the sweep/slice module 214 took multiple images slices across a sweep of sequentially changing elevational angles. Each image slice may be taken while the transducer array is held steady relative to a reference position (typically flat against the habitus of the patient) and in a consistent location with a consistent orientation. Each image slice in memory 1002 is a data file that includes the captured ultrasound image data and the information as the order in which the image slice occurred in the sweep and its relative elevational angle as compared to the other image slices in memory. The image processor 215 may use the sequential order of the image data and the elevational angle of that image slice to join together the image slices into a 3D model of anatomical target and the image space captured by the sweep/slice function.

As such, the systems and method described herein generate a 3D model 1012 that is a 3D representation of the entire imaging region which was imaged by the device. This allows the system to generate a 3D model of an entire imaging target 1014. The ultrasound image data 1004 (the sequence of image slices) is a set of electronic information that the image processor 215 can use to generate for each ultrasound image, data such as the position of visible elements and the angle of imaging. In one embodiment, the image processor 1008 analyzes the ultrasound image data 1004 by applying a polar coordinate reference frame to the ultrasound image data 1004.

Typically, the polar coordinate reference frame includes the imaging angle, which is the angle between an elevational dimension of the 2D transducer array and the direction of ultrasonic wave travel. The polar coordinate reference frame also records the distance between a detected imaging target and the 2D transducer array. This data is typical to create an image from the ultrasound data collect by the transducer. In any case, by applying the polar coordinate reference frame to each image in the series of images and converting the polar coordinates to cartesian coordinates, the system can construct a 3D model of the imaging target. The system can then display, to a user, a view of the imaging target from a point of view other than the point of view at which the target was originally imaged.

In one embodiment, applying the polar coordinate reference frame involves determining the distance from the transducer array of each element in the image, and the angle of imaging for each element in the image. The image processor 1008 processes the ultrasound image data 1004 with the applied polar coordinate reference frame, and using known methods such as generative Al, converts the polar coordinates into cartesian coordinates. The image processor then joins (stitches) together each of the images in the stream of ultrasound images so that a single 3D image model is created. This allows the processor 1008 to determine the relative position of elements in one image to elements in the other images in 3D space. The processor 1008 takes the converted cartesian coordinate information and generates a data set which allows the handheld computing device 1010 to display a representation of the three-dimensional imaging target 1014. As will be described in Figure 13, this 3D model 1012 can be displayed from multiple angles, including angles other than the angle of imaging. Techniques for joining images are known and any suitable image processing technique for joining such images may be applied. It will be understood by those of skill in the art, that the systems and methods described herein build the 3D module from 2D image slices that were swept through a known arc of elevational angle with a known progression in the arc from image slice to image slice. This facilitates the development of the 3D model from the 2D image slices.

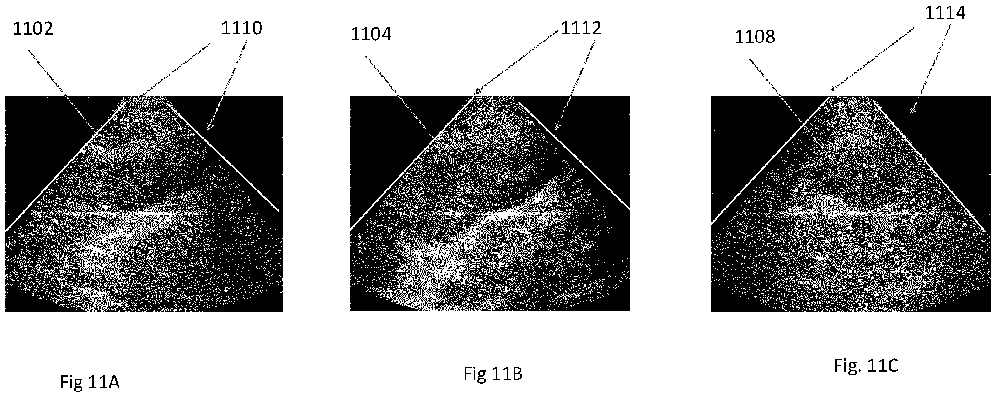

FIGS 11A, 11B and 11C depict examples of individual multislices collected at different points during the scan. FIG. 11A depicts an image taken at an earlier portion of the scan, the imaging target 1102 is near the center of the image. In the depicted example, the imaging target 1102 is a kidney. The borders 1110 depict the outer edges of the region scanned by the ultrasound device. FIG. 11B depicts an ultrasound image taken at a middle portion of the scan, the imaging target 1104 is a larger cross section of a kidney seen in the center of the image. The borders 1112 depict the outer edges of the regions scanned by the ultrasound device. FIG. 11C depicts an ultrasound image taken at a later portion of the scan, the imaging target 1108 is near the center of the image. In this example the imaging target 1108 is a smaller cross section of a kidney. By scanning the target organ from the position in Figure 11A to the position of Figure 11C, the ultrasound device is able to collect ultrasound image data across the entire target organ. The ultrasound device uses beamforming to alter the imaging angle produced generated by the two-dimensional array to scan across the target organ from the position depicted in Figure 8A to the position depicted in Figure 8C. By scanning across the target organ and recording the changes in the detected portion of the target organ, as can be seen between the differences between elements 1102 1104 and 1108, the ultrasound device generates a three-dimensional model based on the ultrasound image data collected during the scan.

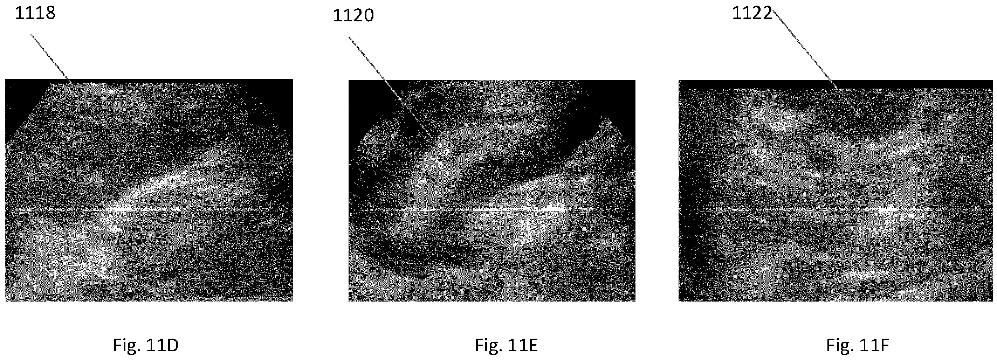

FIGS. 11D, 11E and 11F depict frames of a 3D model generated from ultrasound image data collected in the scan depicted in FIGS. 11A, 11B and 11C. FIG. 11D depicts a 3D model of the ultrasound scan of the imaging target from an earlier portion of the scan. In particular, Figure 11D depicts a cross section of the imaging target 1118. Figure 11E depicts a frame from the 3D model of the ultrasound scan, in particular a model of a larger cross section of the imaging target 1120. Figure 11F depicts a 3D model of a ultrasound scan of the target organ from a later portion of the scan. In particular, it depicts a smaller cross section of the imaging target 1122. This cross section roughly corresponds to the cross section of the imaging target 1108 in Figure 11C.

The 3D model shown in FIGS. 11D, 11E and 11F uses the same viewing angle as the imaging angle used to take the ultrasound scans of Figures 11A 11B and 11C. As can be seen in FIGS. 11A through 11F, viewing the 3D model from the same viewing angle as the imaging angle results in images that roughly correspond to the ultrasound images generated by scanning the target organ. Further, by scanning the target organ across a region as depicted in FIGS. 11A 11B and 11C. The ultrasound system is able to generate a three-dimensional model of the target organ. This allows the system to display a scan of the target organ from an angle other than the angle used for imaging.

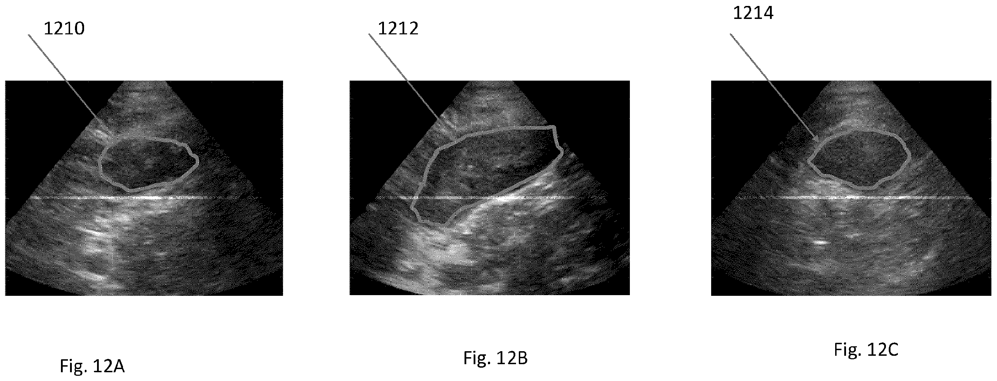

Figures 12A 12B and 12C depict different frames from a scan of a target organ by the ultrasound device. In order to generate a 3D model of the target organ from the scan, the ultrasound device analyzes the ultrasound image data produced by the scan to identify the organ within the ultrasound images. In particular. In Figure 12A, the border of the target organ 1210 is outlined to indicate where the ultrasound device would determine the cross-sectional shape of the target organ is at that point. Figure 12B depicts the outline of the target organ 1212, with a noticeably different shape to the cross-sectional area of the target organ. Figure 12C depicts a cross-sectional border of the target organ 1214, which is similar to that of Figure 12A. The ultrasound system would analyze the changes in the target organs cross-sectional shape from the point in Figure 12A to the point in Figure 12C to determine the three-dimensional shape of the target organ throughout that same region. This allows the ultrasound system to generate a three-dimensional model of the target organ and enable viewing of the target organ from an angle other than the angle of imaging.

FIG. 13 depicts a still image from an ultrasound scan of a target organ using the methods described herein, and three still images from a three-dimensional (3D) model generated based on the ultrasound image slice data produced by the scan. The top left image 1302 is a still frame from an ultrasound scan of the target organ. The bottom left image 1312 is a three-dimensional (3D) model produced by the ultrasound image data generated by the scan depicted in 1302, from the same angle 1312 used to image the target organ. The top right image 1308 depicts the same target organ from the scan 1302, but viewed from a side angle, 1318. Further, the bottom right image 1310 depicts the same target organ from the ultrasound scan 1302 viewed from a top-down angle 1314. By generating a three-dimensional (3D) model of the target organ, the ultrasound systems described herein are able to simulate viewing the target organ from multiple angles, including angles other than the angle of imaging. Thus, the systems described herein can include a user interface feature that allows the user to quickly see the target organ from various angles, which may be desirable, without having to reposition the ultrasound device.

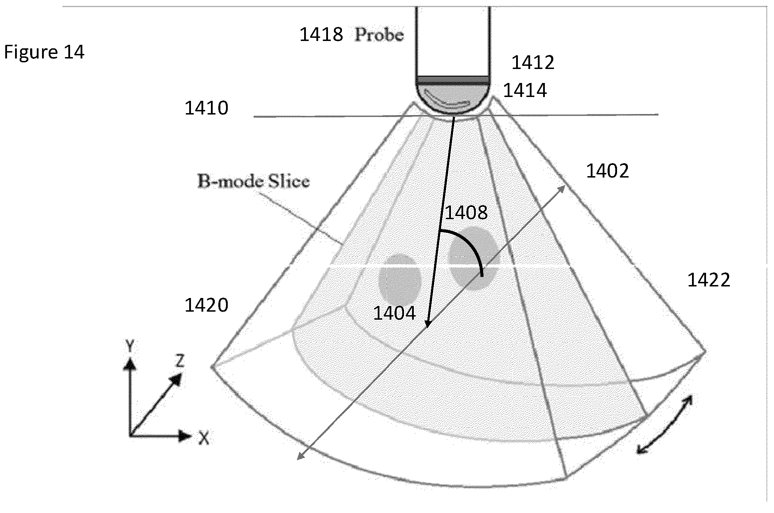

FIG. 14 depicts a handheld ultrasound probe 1418 in contact with a subject 1410 and imaging into the subject to reach a target organ which is along axis 1402. The ultrasound probe 1418 produces the ultrasound ultrasonic signals 1404 from a flat 2D array 1412 and propagated through the subject 1410 from the lens 1414. The target organ is along axis 1402 which is parallel to an elevational dimension of the array 1412. When scanning the target organ, the ultrasound device will record within the ultrasound image data the distance the ultrasonic signals 1404 travelled from the probe to the target organ and the angle of imaging 1408. In the depicted example, the ultrasonic signals 1404 have been controlled with beam steering to travel along plane 1420, representing an outermost image in the series of scanned images. The probe 1418 will scan along the elevational dimension of the array 1412 taking a series of images from plane 1420, to plan 1422. In each image, elements detected by ultrasonic signals 1404 will be recorded as ultrasound image data where the distance travelled by the ultrasonic signals 1404 and the imaging angle 1408 of each element may be calculated. It will be clear to one skilled in the art that elements within the subject other than those along axis 1402 will also be recorded as ultrasound image data where their distance and imaging angle may be calculated. This allows the ultrasound system to apply a polar coordinate reference frame to the ultrasound image data collected during the scan. This polar coordinate reference frame can be converted into Cartesian coordinates in order to generate a three-dimensional model of the target organ imaged during the scan.

The systems and methods described herein reference circuits, processors, memory, CPUs and other devices, and those of skill in the art will understand that these embodiments are examples, and the actual implementation of these circuits may be carried out as software modules running on microprocessor devices and may comprise firmware, software, hardware, or any combination thereof that is configured to perform as the systems and processes described herein.

Further, some embodiments may also be implemented by the preparation of application-specific integrated circuits (ASICs) or by interconnecting an appropriate network of conventional component circuits. To illustrate this interchangeability of hardware and software, various illustrative components, blocks, modules, circuits, and steps have been described herein generally in terms of their functionality. Whether such functionality is implemented as hardware or software depends upon the application and design constraints imposed on the overall system. Skilled artisans may implement the described functionality in varying ways for each particular application, but such implementation decisions should not be interpreted as causing a departure from the embodiments described herein.

Accordingly, it will be understood that the invention is not to be limited to the embodiments disclosed herein but are to understood by the claims and the embodiments covered by the claims and which include, but are not limited to:

## Claims

1. An ultrasound system comprising;
a handheld ultrasound imaging device (102) including;
a flat two-dimensional (2D) array (230) of micromachined ultrasound transducers (MUTs), and
a processor configured to control the 2D array (230) to take a series of ultrasound images along an elevational direction of the 2D array (230) where each image is taken at a different angle relative to an axis parallel to the elevational direction of the array by beam steering ultrasonic signals produced by the MUTs, and
store the series of ultrasound images in a memory as a series of ultrasound image data, and
a handheld computing device (108) coupled to the handheld ultrasound imaging device (102) having a memory capable of storing ultrasound image data, and
wherein the handheld ultrasound imaging device (102) is configured to transmit ultrasound image data to the handheld computing device (108) and store the data in the memory
and wherein
the handheld computing device (108) includes an image processor for analyzing the ultrasound image data stored in memory to reconstruct a three-dimensional (3D) model of an imaging target,
a sweep module (214) and a video module (203) operating in cooperation to present the ultrasound image data that is stored to reconstruct the three-dimensional (3D) model to simulate a rocking maneuver of the handheld ultrasound imaging device (102) to display the ultrasound images sequentially from an image collected at a first angle to an image collected at a furthest angle and from that image collected at that furthest angle back to the image collected at that first angle, and
a display for displaying the ultrasound image data with the simulated rocking maneuver as a video, or a cine.

2. The system of claim 1 where the image processor analyzes the ultrasound image data stored in memory to identify an angle of imaging for each image in the series of ultrasound images, and
identify elements of the imaging target within the ultrasound image.

3. The system of claim 1 where the image processor analyzes the ultrasound image data stored in memory to apply a polar coordinate reference frame to the ultrasound image data and converts the ultrasound image data into image data in cartesian coordinates.

4. The system of claim 2 and 3 where the image processor applying a polar coordinate reference frame comprises comparing an imaging angle of each image and a distance from each element identified in the image and comparing the corresponding values in each image in the series of ultrasound images.

5. The system of claim 1 where the image processor is configured to select an image from the series of ultrasound images wherein the selected image is at the center of a sweep of images slices and generated from an ultrasound beam directed perpendicular to a face of the transducer array (230) and designates it as a key-frame.

6. The system of claim 5 where the image processor is configured to display the key frame as a default still image, or as a central frame in a displayed video or cine.

7. The system of claim 5 further comprising a user interface to allow the user to select a different image from the series of ultrasound images and designate it as the key-frame to allow the user to select an image to present a pathology that the user wants visualized.

8. The system of claim 1 wherein the image processor is configured to join together the series of ultrasound images taken along an elevational direction of the 2D array (230) to reconstruct the three-dimensional (3D) model of the imaging target as a model having data of the imaging target throughout a three-dimensional space.

9. The system of claim 1 further comprising a single button on the ultrasound imaging device to activate the processor to control the array.

10. The system of claim 1 further comprising a user interface configured to display a bounced image comprising a sequence of brightness mode images presented so that features of interest will from time to time appear more brightly within some of the images.

11. A method of imaging an organ comprising;
using a handheld ultrasound imaging device including a two-dimensional (2D) array of micromachined ultrasound transducers (MUTs), a processor capable of controlling the array, and
using a handheld computing device including an image processor, a sweep module, a video module, and a display, and
using the handheld ultrasound imaging device to take a series of ultrasound images along an elevational dimension of the 2D array where each image in the series of ultrasound images has a different angle of imaging relative to an axis parallel to the elevational dimension of the array by beam steering ultrasonic signals produced by the MUTs, and
having the processor of the ultrasound imaging device process each image in the series of ultrasound images to generate ultrasound image data and transmit the ultrasound image data to the handheld computing device,
having the image processor process the ultrasound image data to generate a three-dimensional (3D) model of an imaging target imaged by the series of ultrasound images,
having the sweep module and the video module operate in cooperation to present the ultrasound image data used to generate the three-dimensional (3D) model with a simulated rocking maneuver of the handheld ultrasound imaging device to display the ultrasound images sequentially from an image collected at a first angle to a furthest image collected at a furthest angle and from that image collected at that furthest angle back to the image collected at that first angle, and
having the display display the ultrasound image data with the simulated rocking maneuver as a video, or a cine to the user.

12. The method of claim 11 where processing the ultrasound image data comprises identifying the angle of imaging of each image in the series of ultrasound images, and identifying elements of an imaging target within the ultrasound image.

13. The method of claim 11 where processing the ultrasound image data comprises applying a polar coordinate reference frame to the ultrasound image data and converting the ultrasound image data into image data in cartesian coordinates.

14. The method of claims 12 and 13 where applying a polar coordinate reference frame comprises comparing an imaging angle of each image and a distance from each element identified in the image and comparing the corresponding values in each image in the series of ultrasound images.

15. The method of claim 11 where displaying the ultrasound image data includes selecting an image from the series of ultrasound images that is at the center of a sweep of images and is generated from an ultrasound beam directed perpendicular to a face of the transducer array and designating the selected image as a key-frame.

## Patentansprüche

1. Ultraschallsystem, umfassend;
eine tragbare Ultraschallbildgebungsvorrichtung (102), beinhaltend;
ein flaches zweidimensionales (2D) Array (230) von mikromechanischen Ultraschallwandlern (MUT) und
einen Prozessor, der konfiguriert ist, um das 2D-Array (230) zu steuern, um eine Reihe von Ultraschallbildern entlang einer Elevationsrichtung des 2D-Arrays (230) aufzunehmen, bei dem jedes Bild in einem unterschiedlichen Winkel relativ zu einer Achse parallel zu der Elevationsrichtung des Arrays durch Strahllenkung von Ultraschallsignalen aufgenommen wird, die durch die MUT erzeugt werden, und die Reihe von Ultraschallbildern in einem Speicher als eine Reihe von Ultraschallbilddaten zu speichern, und
eine tragbare Rechenvorrichtung (108), die an die tragbare Ultraschallbildgebungsvorrichtung (102) gekoppelt ist, die einen Speicher aufweist, der in der Lage ist, Ultraschallbilddaten zu speichern, und
wobei die tragbare Ultraschallbildgebungsvorrichtung (102) konfiguriert ist, um Ultraschallbilddaten an die tragbare Rechenvorrichtung (108) zu übertragen und die Daten in dem Speicher zu speichern, und wobei
die tragbare Rechenvorrichtung (108) einen Bildprozessor zum Analysieren der in dem Speicher gespeicherten Ultraschallbilddaten beinhaltet, um ein dreidimensionales (3D) Modell eines Bildgebungsziels zu rekonstruieren,
wobei ein Sweep-Modul (214) und ein Videomodul (203) zusammenarbeiten, um die gespeicherten Ultraschallbilddaten darzustellen, um das dreidimensionale (3D) Modells zu rekonstruieren, um ein Schaukelmanöver der tragbaren Ultraschallbildgebungsvorrichtung (102) zu simulieren, um die Ultraschallbilder nacheinander von einem Bild, das in einem ersten Winkel erfasst wurde, bis zu einem Bild, das in einem maximalen Winkel erfasst wurde, und von diesem Bild, das in diesem maximalen Winkel erfasst wurde, zurück zu dem Bild, das in diesem ersten Winkel erfasst wurde, anzuzeigen, und
eine Anzeige zum Anzeigen der Ultraschallbilddaten mit dem simulierten Schaukelmanöver als Video oder Film.

2. System nach Anspruch 1, bei dem der Bildprozessor die in dem Speicher gespeicherten Ultraschallbilddaten analysiert, um für jedes Bild in der Reihe von Ultraschallbildern einen Bildgebungswinkel zu identifizieren und
Elemente des Bildgebungsziels innerhalb des Ultraschallbildes zu identifizieren.

3. System nach Anspruch 1, bei dem der Bildprozessor die in dem Speicher gespeicherten Ultraschallbilddaten analysiert, um einen Polarkoordinaten-Bezugsrahmen auf die Ultraschallbilddaten anzuwenden, und die Ultraschallbilddaten in Bilddaten in kartesischen Koordinaten umwandelt.

4. System nach Anspruch 2 und 3, bei dem das Anwenden eines Polarkoordinaten-Bezugsrahmens durch den Bildprozessor Vergleichen eines Bildgebungswinkels jedes Bildes und eines Abstands von jedem in dem Bild identifizierten Element und Vergleichen der entsprechenden Werte in jedem Bild in der Reihe von Ultraschallbildern umfasst.

5. System nach Anspruch 1, bei dem der Bildprozessor konfiguriert ist, um ein Bild aus der Reihe von Ultraschallbildern auszuwählen, wobei das ausgewählte Bild in der Mitte eines Sweeps von Bild-Slices liegt und aus einem Ultraschallstrahl generiert wird, der senkrecht zu einer Fläche des Wandlerarrays (230) gerichtet ist, und es als Schlüsselbild bezeichnet.

6. System nach Anspruch 5, bei dem der Bildprozessor konfiguriert ist, um das Schlüsselbild als Standard-Standbild oder als zentrales Einzelbild in einem angezeigten Video oder Film anzuzeigen.

7. System nach Anspruch 5, ferner umfassend eine Benutzeroberfläche, um es dem Benutzer zu ermöglichen, ein anderes Bild aus der Reihe von Ultraschallbildern auszuwählen und es als Schlüsselbild zu bezeichnen, um dem Benutzer zu ermöglichen, ein Bild auszuwählen, um eine Pathologie darzustellen, die der Benutzer visualisieren möchte.

8. System nach Anspruch 1, wobei der Bildprozessor konfiguriert ist, um die Reihe von Ultraschallbildern, die entlang einer Elevationsrichtung des 2D-Arrays (230) aufgenommen wurde, miteinander zu verbinden, um das dreidimensionale (3D) Modell des Bildgebungsziels als ein Modell zu rekonstruieren, das Daten des Bildgebungsziels überall in einem dreidimensionalen Raum aufweist.

9. System nach Anspruch 1, ferner umfassend eine einzelne Taste an der Ultraschallbildgebungsvorrichtung, um den Prozessor zu aktivieren, um das Array zu steuern.

10. System nach Anspruch 1, ferner umfassend eine Benutzeroberfläche, die konfiguriert ist, um ein reflektiertes Bild anzuzeigen, umfassend eine Sequenz von Helligkeitsmodusbildern, die so dargestellt wird, dass relevante Merkmale von Zeit zu Zeit innerhalb einiger der Bilder heller erscheinen.

11. Verfahren zur Bildgebung eines Organs, umfassend;
Verwenden einer tragbaren Ultraschallbildgebungsvorrichtung, die ein zweidimensionales (2D) Array von mikromechanischen Ultraschallwandlern (MUT) beinhaltet, wobei ein Prozessor in der Lage ist, das Array zu steuern, und
Verwenden einer tragbaren Rechenvorrichtung, die einen Bildprozessor, ein Sweep-Modul, ein Videomodul und eine Anzeige beinhaltet, und
Verwenden der tragbaren Ultraschallbildgebungsvorrichtung, um eine Reihe von Ultraschallbildern entlang einer Elevationsdimension des 2D-Arrays aufzunehmen, bei dem jedes Bild in der Reihe von Ultraschallbildern einen unterschiedlichen Bildgebungswinkel relativ zu einer Achse parallel zu der Elevationsdimension des Arrays aufweist, durch Strahllenkung von Ultraschallsignalen, die durch die MUT erzeugt werden, und Veranlassen, dass der Prozessor der Ultraschallbildgebungsvorrichtung jedes Bild in der Reihe von Ultraschallbildern verarbeitet, um Ultraschallbilddaten zu generieren und die Ultraschallbilddaten an die tragbare Rechenvorrichtung zu übertragen,
Veranlassen, dass der Bildprozessor die Ultraschallbilddaten verarbeitet, um ein dreidimensionales (3D) Modell eines durch die Reihe von Ultraschallbildern abgebildeten Bildgebungsziels zu generieren, Veranlassen, dass das Sweep-Modul und das Videomodul zusammenarbeiten, um die Ultraschallbilddaten darzustellen, die verwendet werden, um das dreidimensionale (3D) Modell mit einem simulierten Schaukelmanöver der tragbaren Ultraschallbildgebungsvorrichtung zu generieren, um die Ultraschallbilder nacheinander von einem Bild, das in einem ersten Winkel erfasst wurde, bis zu einem Bild, das in einem maximalen Winkel erfasst wurde, und von diesem Bild, das in diesem maximalen Winkel erfasst wurde, zurück zu dem Bild, das in diesem ersten Winkel erfasst wurde, anzuzeigen, und Veranlassen, dass die Anzeige die Ultraschallbilddaten mit dem simulierten Schaukelmanöver als Video oder Film für den Benutzer anzeigt.

12. Verfahren nach Anspruch 11, bei dem das Verarbeiten der Ultraschallbilddaten Identifizieren des Bildgebungswinkels jedes Bildes in der Reihe von Ultraschallbildern und Identifizieren von Elementen eines Bildgebungsziels innerhalb des Ultraschallbildes umfasst.

13. Verfahren nach Anspruch 11, bei dem das Verarbeiten der Ultraschallbilddaten Anwenden eines Polarkoordinaten-Bezugsrahmens auf die Ultraschallbilddaten und Umwandeln der Ultraschallbilddaten in Bilddaten in kartesischen Koordinaten umfasst.

14. Verfahren nach Anspruch 12 und 13, bei dem das Anwenden eines Polarkoordinaten-Bezugsrahmens Vergleichen eines Bildgebungswinkels jedes Bildes und eines Abstands von jedem in dem Bild identifizierten Element und Vergleichen der entsprechenden Werte in jedem Bild in der Reihe von Ultraschallbildern umfasst.

15. Verfahren nach Anspruch 11, bei dem das Anzeigen der Ultraschallbilddaten Auswählen eines Bildes aus der Reihe von Ultraschallbildern, die sich in der Mitte eines Sweeps von Bildern befindet und aus einem Ultraschallstrahl generiert wird, der senkrecht zu einer Fläche des Wandlerarrays gerichtet ist, und Bezeichnen des ausgewählten Bildes als Schlüsselbild beinhaltet.

## Revendications

1. Système à ultrasons comprenant ;
un dispositif d'imagerie par ultrasons portable (102) comportant ;
un réseau plat bidimensionnel (2D) (230) de transducteurs à ultrasons micro-usinés (MUT), et
un processeur configuré pour commander le réseau 2D (230) afin de prendre une série d'images ultrasonores le long d'une direction d'élévation du réseau 2D (230), où chaque image est prise à un angle différent par rapport à un axe parallèle à la direction d'élévation du réseau par déviation de faisceau de signaux ultrasonores produits par les MUT, et
stocker la série d'images ultrasonores dans une mémoire sous forme de série de données d'images ultrasonores, et
un dispositif informatique portable (108) couplé au dispositif d'imagerie par ultrasons portable (102) ayant une mémoire capable de stocker des données d'images ultrasonores, et
dans lequel le dispositif d'imagerie par ultrasons portable (102) est configuré pour transmettre des données d'images ultrasonores au dispositif informatique portable (108) et stocker les données dans la mémoire
et dans lequel
le dispositif informatique portable (108) comporte un processeur d'images destiné à analyser les données d'images ultrasonores stockées en mémoire afin de reconstruire un modèle tridimensionnel (3D) d'une cible d'imagerie,
un module de balayage (214) et un module vidéo (203) fonctionnant en coopération pour présenter les données d'images ultrasonores qui sont stockées afin de reconstruire le modèle tridimensionnel (3D) pour simuler une manœuvre de bascule du dispositif d'imagerie par ultrasons portable (102) afin d'afficher les images ultrasonores de manière séquentielle, depuis une image collectée à un premier angle vers une image collectée à un angle le plus éloigné, et depuis cette image collectée à cet angle le plus éloigné vers l'image collectée à ce premier angle, et
un affichage destiné à afficher les données d'images ultrasonores avec la manœuvre de bascule simulée sous forme de vidéo ou de film.

2. Système selon la revendication 1 où le processeur d'images analyse les données d'images ultrasonores stockées en mémoire afin d'identifier un angle d'imagerie pour chaque image de la série d'images ultrasonores, et
identifier les éléments de la cible d'imagerie au sein de l'image ultrasonore.

3. Système selon la revendication 1 où le processeur d'images analyse les données d'images ultrasonores stockées en mémoire pour appliquer un repère de coordonnées polaires aux données d'images ultrasonores et convertit les données d'images ultrasonores en données d'images en coordonnées cartésiennes.

4. Système selon la revendication 2 et 3 où le processeur d'images appliquant un repère de coordonnées polaires comprend la comparaison d'un angle d'imagerie de chaque image et d'une distance à partir de chaque élément identifié dans l'image et la comparaison des valeurs correspondantes dans chaque image de la série d'images ultrasonores.

5. Système selon la revendication 1 où le processeur d'images est configuré pour sélectionner une image à partir de la série d'images ultrasonores, dans lequel l'image sélectionnée est au centre d'un balayage de tranches d'images et est générée à partir d'un faisceau ultrasonore dirigé perpendiculairement à une face du réseau de transducteurs (230), et la désigne en tant que trame clé.

6. Système selon la revendication 5 où le processeur d'images est configuré pour afficher la trame clé en tant qu'image fixe par défaut, ou en tant que trame centrale dans une vidéo ou un film affiché.

7. Système selon la revendication 5 comprenant en outre une interface utilisateur permettant à l'utilisateur de sélectionner une image différente à partir de la série d'images ultrasonores et de la désigner en tant que trame clé pour permettre à l'utilisateur de sélectionner une image afin de présenter une pathologie que l'utilisateur souhaite visualiser.

8. Système selon la revendication 1 dans lequel le processeur d'images est configuré pour assembler la série d'images ultrasonores prises le long d'une direction d'élévation du réseau 2D (230) afin de reconstruire le modèle tridimensionnel (3D) de la cible d'imagerie sous forme de modèle ayant des données de la cible d'imagerie dans un espace tridimensionnel.

9. Système selon la revendication 1 comprenant en outre un seul bouton sur le dispositif d'imagerie par ultrasons pour activer le processeur afin de commander le réseau.

10. Système selon la revendication 1 comprenant en outre une interface utilisateur configurée pour afficher une image réfléchie comprenant une séquence d'images en mode de luminosité présentées de telle sorte que les caractéristiques d'intérêt apparaissent, de temps à autre, plus lumineuses au sein de certaines des images.

11. Procédé d'imagerie d'un organe comprenant ;
l'utilisation d'un dispositif d'imagerie par ultrasons portable comportant un réseau bidimensionnel (2D) de transducteurs ultrasonores micro-usinés (MUT), un processeur capable de commander le réseau, et
l'utilisation d'un dispositif informatique portable comportant un processeur d'images, un module de balayage, un module vidéo et un affichage, et
l'utilisation du dispositif d'imagerie par ultrasons portable pour prendre une série d'images ultrasonores le long d'une dimension d'élévation du réseau 2D, où chaque image de la série d'images ultrasonores a un angle d'imagerie différent par rapport à un axe parallèle à la dimension d'élévation du réseau par déviation de faisceau de signaux ultrasonores produits par les MUT, et
le fait de faire traiter, par le processeur du dispositif d'imagerie par ultrasons, chaque image de la série d'images ultrasonores afin de générer des données d'images ultrasonores et de transmettre les données d'images ultrasonores au dispositif informatique portable,
le fait de faire traiter, par le processeur d'images, les données d'images ultrasonores pour générer un modèle tridimensionnel (3D) d'une cible d'imagerie imagée par la série d'images ultrasonores, le fait de faire fonctionner le module de balayage et le module vidéo en coopération afin de présenter les données d'images ultrasonores utilisées pour générer le modèle tridimensionnel (3D) avec une manœuvre de bascule simulée du dispositif d'imagerie par ultrasons portable afin d'afficher les images ultrasonores de manière séquentielle, depuis une image collectée à un premier angle vers une image la plus éloignée collectée à un angle le plus éloigné, et depuis cette image collectée à cet angle le plus éloigné vers l'image collectée à ce premier angle, et
le fait de faire afficher, par l'affichage, les données d'images ultrasonores avec la manœuvre de bascule simulée sous forme de vidéo ou de film à l'utilisateur.

12. Procédé selon la revendication 11 où le traitement des données d'images ultrasonores comprend l'identification de l'angle d'imagerie de chaque image de la série d'images ultrasonores, et l'identification d'éléments d'une cible d'imagerie au sein de l'image ultrasonore.

13. Procédé selon la revendication 11 où le traitement des données d'images ultrasonores comprend l'application d'un repère de coordonnées polaires aux données d'images ultrasonores et la conversion des données d'images ultrasonores en données d'image en coordonnées cartésiennes.

14. Procédé selon les revendications 12 et 13 où l'application d'un repère de coordonnées polaires comprend la comparaison d'un angle d'imagerie de chaque image et d'une distance à partir de chaque élément identifié dans l'image et la comparaison des valeurs correspondantes dans chaque image de la série d'images ultrasonores.

15. Procédé selon la revendication 11 où l'affichage des données d'images ultrasonores comporte la sélection d'une image à partir de la série d'images ultrasonores qui est au centre d'un balayage d'images et qui est générée à partir d'un faisceau ultrasonore dirigé perpendiculairement à une face du réseau de transducteurs et la désignation de l'image sélectionnée en tant que trame clé.
